# EUROPEAN PATENT SPECIFICATION

(11) **EP 3 957 341 B1**
(45) Date of publication and mention of the grant of the patent: **13.03.2024**
(21) Application number: 21192010.3
(22) Date of filing: 18.08.2021
(51) Int. Cl.: A61L 31/12, A41D 13/11, A61F 9/00

(54) **MULTILAYER COPPER-BASED ZEOLITE FIBER MEDICAL MATERIAL, PROTECTIVE EQUIPMENT AND MANUFACTURING METHOD**
MEHRSCHICHTIGES MEDIZINISCHES ZEOLITHFASERMATERIAL AUF KUPFERBASIS, SCHUTZAUSRÜSTUNG UND HERSTELLUNGSVERFAHREN
MATÉRIAU MÉDICAL DE FIBRES DE ZÉOLITE MULTICOUCHES À BASE DE CUIVRE, ÉQUIPEMENT DE PROTECTION ET PROCÉDÉ DE FABRICATION

(30) Priority: 19.08.2020 CN 202010839473
(43) Date of publication of application: 23.02.2022
(73) Proprietor: Zhejiang University, Hangzhou City, Zhejiang 310058 (CN); Hangzhou Zeo-Innov Life Technology Co., Ltd., Hangzhou, Zhejiang Province 310018 (CN)
(72) Inventor: FAN, Jie, Hangzhou (CN); XIAO, Liping, Hangzhou (CN); YU, Lisha, Hangzhou (CN); LI, Dan, Hangzhou (CN); SHI, Yifeng, Hangzhou (CN); YAO, Hangping, Hangzhou (CN)
(74) Representative: Chung, Hoi Kan

(56) References cited:
- CN-A- 111 227 345
- US-A1- 2008 295 843
- US-A1- 2012 082 711

## Description

### Technical Field

This disclosure relates to the field of sanitary appliances used for epidemic prevention, in particular to a medical material with sufficiently low cytotoxicity, high antibacterial and effective antiviral performance. Specially, it can be used to prepare biosafety medical materials and medical protective equipment for killing SARS-CoV-2.

### Background

The emergence of new and sudden infectious diseases has caused great harm to human health, social politics, and economy. Coronavirus is a large family of viruses that can cause infectious diseases such as colds, Middle East Respiratory Syndrome (MERS), Severe Acute Respiratory Syndrome (SARS) and Corona Virus Disease 2019 (COVID-19). Among them, main modes of severe acute respiratory syndrome coronavirus 2 (SARS-CoV-2) transmission are: direct transmission, aerosol transmission, contact transmission, etc. Therefore, wearing personal protective equipment is one of the most effective protective measures to prevent virus infection.

For dangerous bacteria and viruses, a small amount of contact/inhalation is dangerous. In fact, medical personnel and epidemic prevention personnel are often faced with patients and contagious environments in their work. In case of improper protection, they are easy to be infected.

At present, commonly used medical masks are composed of three layers, in which both the outer layer and the inner layer are made of non-woven fabric, and the middle layer is made of melt-blown non-woven fabric. The outer layer of the mask is designed to be anti-droplet; the middle layer is the core functional layer for filtering out airborne droplets, particles or bacteria; and the inner layer mainly absorbs moisture. Masks use the principle of filtration to physically block viruses, and they can block some pathogens such as viruses and bacteria, thereby reducing the risk of virus infection to a certain degree. However, it is unavoidable that there are a few viruses and bacteria attached to the surface of the mask. Studies have confirmed that SARS-CoV-2 captured or trapped in a mask can survive for up to 7 days. The viable virus attached to the surface of the mask is likely to become a source of cross-infection, allowing the virus to spread again. Therefore, how to kill the virus residue on the surface of the mask or the filter layer is a key issue in the entire epidemic prevention system.

According to the type of mask use, masks are categorized as civilian masks and medical masks. Medical masks are mainly divided into three categories, namely ordinary medical masks, medical surgical masks and medical protective masks (Sun Xingchun, et al. Patent Technology Development of Medical Protective Masks. New Material Industry, 2020(2): 13-15). Medical masks use the filtering effect of masks to block bacteria and viruses, focusing on the protective function. Therefore, the national standards or industry standards for medical masks have requirements for filtration efficiency, and the requirement order is ordinary medical masks < medical surgery masks < medical protective masks, which means the filtering effect of medical protective masks is the best. However, there is not antibacterial and antiviral technical standards set for the above three types of medical masks. Among the medical masks, there are biological safety standards about cytotoxicity (cytotoxicity should not be greater than 2) set for the ordinary medial masks and medical surgical masks, but no for the medical protection masks.

Due to the short wearing time and low protection requirements, there is no regulation about cytotoxicity for civilian masks. Ordinary medical masks or medical surgical masks must comply with the cytotoxicity regulations in the industry standards: the pharmaceutical industry standard YY/T0969-2013 "Disposable Medical Face Mask" and YY 0469-2011 "Medical Surgical Mask" issued by the China Food and Drug Administration. These standards stipulate that the cytotoxicity of the mask should not be greater than 2, which means that no more than 50% of cell inhibition can be observed (that is, the cell survival rate is greater than 50%). In addition, cytotoxicity must be strictly implemented in accordance with the China National Standard GB/T 16886.5-2017/ISO 10993-5:2009 "Biological Evaluation of Medical Devices--Part 5: Tests for in tro cytotoxicity". Also, medical protective masks must comply with the Standard GB19083-2010 "Technical Requirements for Medical Protective Masks", whose important indicators are mainly filtration efficiency and inhalation resistance, and no regulations on biological safety issues related to cytotoxicity. In the prior art, medical protective masks that meet the N95 standard adopt passive protection technology (the filtration efficiency against particles with aerodynamic physical diameter of 0.075 µm±0.020 µm can reach more than 95%), which only provides pure filtration and barrier performance and difficult to guarantee use safety for professionals in medical protection, disease prevention, and epidemic prevention and control of infectious diseases.

With the spread and continuous mutation of SARS-CoV-2, the viral survivability of the virus gets stronger. The sole filtration effect against the virus through the mask cannot meet the protection requirements for SARS-CoV-2. Therefore, higher requirements are put forward for the performance of medical masks, that is, the function of killing bacteria and viruses. Current existing methods for medical masks to achieve better antibacterial and antiviral performance includes: nanosized materials (nanosized zinc oxide, graphene, silver nanoparticles), photocatalyst technology (e.g. titanium dioxide), traditional Chinese medicine, and antibacterial function layers. Currently, the antibacterial and antiviral performance of the market available medical masks are a mixed bag and there are not uniform standards. The current national or industry standards do not set antibacterial and antiviral technical requirements and cytotoxic biological safety requirements for medical masks at the same time. At present, the function of killing bacteria and viruses of medical masks is achieved by destroying the cell membrane/wall of bacteria or viruses. This will also destroy the cell structure of bacteria or viral host cell, making the biosafety evaluation of masks, particularly the cytotoxicity, unsatisfactory, left the masks with lot of cytotoxicity. It can be seen that the prior art obtains the antibacterial and antiviral effects of medical masks at the expense of biological safety, especially for medical protective masks with the highest level of protection. The better the antibacterial and antiviral effects, the greater the cytotoxicity. Therefore, it is impossible to set relevant technical standards for the cytotoxicity of medical protective masks, and it is impossible to set antibacterial and antiviral technical requirements and cytotoxic biological safety requirements for all types of medical masks at the same time.

Copper can kill bacteria and viruses, and can be used for antibacterial and antiviral masks, and it should be loaded on the mask material in a suitable form to ensure the flexibility of the mask and the biological safety. Among them, biological safety is one of the most important indicators of medical masks. It refers to cytotoxicity, skin irritation, and delayed hypersensitivity reactions, among which cytotoxicity is the most critical technical indicator for biosafety evaluation. Cytotoxicity refers to the destruction of cell structure by extracellular chemicals, which ultimately leads to cell apoptosis or necrosis and then reduces the survival rate of cells in in vitro experiments.

At present, the application of copper in medical mask materials has two major safety hazards based on its cytotoxicity: 1) Although copper (especially divalent copper ions) has a good effect on killing bacteria and viruses, it has strong cytotoxicity (Chen Demin. Evaluation of the cytotoxicity of nine metal ions[J]. Chinese Journal of Dental Materials and Devices, 1993, 000(002): 12-13.). The cytotoxicity of copper is positively correlated with antibacterial and antiviral performance, that is, the higher the copper content, the better the antibacterial and antiviral performance, while the greater the cytotoxicity. When the concentration of divalent copper ions in the solution is 0.5 ppm or 5 ppm, the cytotoxicity is level 1 and it does not have antibacterial and antiviral functions; when the concentration of divalent copper ions in the solution is 10 ppm, it has certain antibacterial and antiviral functions, and the cytotoxicity thereof is level 4, much higher than the regulations about cytotoxicity in the industry standard. This cannot meet the requirements of medical masks or medical surgical masks. 2) In the process of wearing a mask, when droplets or aerosols pass through the mask layer with copper elements, the aerosols are very easy to exchange substances with the copper elements in the fiber layer of the mask (including ion exchange) as the copper ions are extremely easy to dissolve in the aqueous solution. Because of this, there is a risk that the copper element enters the mouth and nose, which means wearing a mask with copper element for a long time will cause certain damage to the body, especially do harm to the medical staff and epidemic prevention personnel who have been wearing the mask.

It can be seen that the low content of copper element cannot achieve the effect of antibacterial and antiviral performance. On the contrary, in order to achieve efficient antibacterial and antiviral performance, high content of copper element will be needed and cytotoxicity must be sacrificed, as a result the relevant regulations in the medical mask industry standard cannot be met. (See the relationship between the amount of copper ions, cytotoxicity and antiviral performance in Comparative Examples 1-4 in Table 1 of the specification).

Currently, there are three main forms of copper, cupric compound, and cuprous compound for the copper elements used in the manufacturing of masks. These three forms have inherent defects in the manufacturing of medical masks or medical surgical masks: (1) In the form of nanosized and microsized copper particles, copper wires, etc. Although the cytotoxicity of copper is lower than that of cupric ion, the antiviral and antibacterial effect of copper is significantly worse than that of cupric ion, which does not meet the high standards of antibacterial and antiviral protection requirements, and is not suitable for the material of medical or medical surgery mask. (2) In the form of cuprous ion, such as cuprous oxide (risk term R22: harmful if swallowed; R50/53: extremely toxic to aquatic organisms and may have adverse effects on the aquatic environment; safety term S61: avoid release to the environment) and cuprous iodide (safety term S24/25: avoid contact with skin and eyes; hazard category code R36/37/38: irritate the eyes, respiratory system and skin). The cuprous compounds have strong irritation to the eyes, respiratory system and skin and are not suitable for use as materials for medical or medical surgical masks. (3) In the form of cupric compounds, such as cupric sulfate, cupric chloride, cupric nitrate, cupric carbonate, etc. The antiviral and antibacterial effect of cupric ion is better than that of elemental copper, but the cupric ion is very easy to dissolve in water, which will result in a very low content of cupric ion in the final synthesized copper fiber and poor antiviral and antibacterial effect. In order to enhance the antibacterial and antiviral functions, more cupric compounds will be necessary. However, more cupric compounds will increase the cytotoxicity of the mask material at the same time, and does not meet the cytotoxicity regulations in the medical mask industry standard. In addition, if too much cupric compounds are added, large amounts of cupric ions are easy to dissolve in the water vapor on the mask when medical staffs wear masks for a long time, in which circumstance the ions can easily enter the body and endanger their health. At the same time, copper fibers are generally prepared by spinning. Elemental copper has no affinity for fabrics and spinning is difficult. Cuprous compounds generally have color and are not suitable for the spinning of mask fabrics. There are a large amount of loss for cupric compounds during the spinning process, which means it is impossible to prepare a fiber with a high content of cupric ions. At present, the above three forms of copper element cannot achieve both low cytotoxicity and high antibacterial/antiviral performance. Materials containing copper cannot be used for medical masks or medical surgical masks.

The copper-containing materials also include copper ion-modified zeolite materials. Zeolite is a water-containing alkali or alkaline-earth metal aluminosilicate compound. The general formula of the chemical composition of zeolite is: (M)_{2/n}O·xAl₂O₃·ySiO₂·pH₂O, M represents metal ions (such as K⁺, Na⁺, Ca²⁺, Ba²⁺, etc.), n represents the valence of metal ions, and x represents the number of moles of Al₂O₃, y represents the number of moles of SiO₂, and p represents the number of moles of H₂O. The above-mentioned zeolite can be X-type zeolite, Y-type zeolite, A-type zeolite, Chabazite, Mordenite, L-type zeolite, P-type zeolite, and Merlinoite. The charge-balanced metal ions outside the framework of the above-mentioned zeolite can be partially replaced by copper ions through ion exchange, so that the copper ion-modified zeolite has the ability to kill bacteria and viruses, and can control the release of copper ions to a certain extent. A sufficiently high effect of killing bacteria and viruses will inevitably increase the cytotoxicity, and the cytotoxicity level is greater than 2, which does not meet the cytotoxicity regulations of medical masks or medical surgical masks, and cannot be used as a material for medical masks or medical surgical masks. (See the results of Comparative Example 1 and Table 1).

Chinese publication No. 111227345A discloses an antibacterial and antiviral mask and the manufacturing method, which are divided into a surface layer, a core layer and an inner layer from outside in. The surface layer is a hydrophobic polypropylene spunbonded non-woven fabric. The core layer is an antibacterial and antiviral non-woven fabric, which is prepared by mixing ordinary PET short fibers and the antibacterial antiviral short fibers obtained by blending the silver-copper nanosized zeolite material and fibers. Although the mask made by this disclosure has high antiviral activity against SARS, H1N1, H7N9, and H3N2, and high antibacterial activity against *Escherichia coli* and *Staphylococcus aureus,* the instructions also mention that "copper ions are good for attacking cell walls, which allows silver ions to easily invade the cells, and thus jointly attack within the cells". It reveals that copper ions will inevitably destroy the structure of cells in the process of anti-bacterial and anti-virus, and have strong cytotoxicity. As a result, the cytotoxicity problem of masks cannot be guaranteed in order to achieve anti-virus effect. On the other hand, the hydrophobic polypropylene spunbonded non-woven fabric of the outermost layer prevents bacteria and virus droplets from entering the body, the droplets that carry bacteria and viruses still adhere to the outermost layer of the mask and are still active and likely to become a source of cross-infection, allowing the virus to spread again. In other words, the antibacterial and antiviral non-woven fabric of the middle layer can inactivate bacteria and viruses that enter from the outermost layer, but it cannot inactivate the bacteria and viruses in the outermost layer of the mask.

Previous study (Antiviral Research, 2012, 93(2): 225-233.) discloses a copper-containing zeolite cotton textile for inactivating avian influenza H5 subtype viruses. The paper shows that the copper-containing zeolite cotton textile has the effect of inactivating the high pathogenic H5N1 and low pathogenic H5N3 viruses. In the experimental section mentioned in this article, the copper zeolite wool textile was only immersed in the culture solution for 1 min, and the extract was diluted 10 times for the cytotoxicity test of the material. According to the national standard "Medical Device Biological Evaluation Part 5: In Vitro Cytotoxicity Test" GB/T 16886.5-2017/ISO 10993-5:2009, for the extract test (8.2.9) of medical devices, after the sample culture of at least 24 hours, the extract can be used to determine cytotoxicity. The paper shows that the copper zeolite wool textile is cultured with the culture solution for 1 min, and the extract is diluted the extract 10 times for cytotoxicity testing, which is far from meeting the requirements of national standard testing. The conclusion "the copper zeolite cotton textile does not show cytotoxicity". drawn from this testing cannot be used as a reference basis for standard cytotoxicity testing. This textile has strong cytotoxicity in essence and does not meet the cytotoxic biological evaluation standards of YYT 0969-2013 "Disposable Medical Masks" and YY 0469-2011 "Medical Surgical Mask". (See the results of Comparative Example 1 and Table 1 of the present disclosure).

Chinese publication No. 111469498A discloses a medical protective material containing copper ion antibacterial fabric. The medical protective material is composed of a primary filter layer, antibacterial fabric layer, precision filter layer, and a skin-friendly comfort layer sequentially arranged from the outside to the inside. The antibacterial fabric layer is an antibacterial fabric layer containing copper ions. The medical protective materials used as medical masks only need to meet the filtration efficiency and inhalation resistance indicators in the GB/T 19083-2010 "Technical Requirements for Medical Protective Masks" standard. In order to realize the antibacterial and antiviral functions of the medical protective masks, the patent specification mentions that "[copper] ions are positively charged. When the metal ions reach the negatively charged bacterial cell membrane, they can rely on Coulomb attraction to firmly adsorb the cell membrane and further penetrate the cell wall, leading to the rupture of the cell wall and causing cytoplasmic damage. The cytoplasmic outflow hinders the reproduction of bacteria and eventually leads to the death of bacteria." Similarly, the copper ions can also act on the negatively charged cell membranes of human cells to destroy the cell structure, indicating that the medical protective mask has strong cytotoxicity and cannot meet the requirements of biological safety. The statement in the patent specification that "copper is the only metal element that is harmless to the human body, does not pollute the environment, and has good antibacterial performance" is improper. Many authoritative documents have proved that copper has strong cytotoxicity. (① Wataha JC. Effect of cell line on in vitro metal ion cytotoxicity[J]. Dental Materials, 1994, 10(3):156-161; ② Wataha JC, Hanks CT, Craig RG. The in vitro effects of metal cations on eukaryotic cell metabolism.[J]. Journal of Biomedical Materials Research Part A, 2010, 25.). Therefore, the medical protective mask involved in this disclosure obtains antibacterial and antiviral effects at the expense of biological safety, and is not suitable for medical personnel to wear for a long time against SARS-CoV-2, and is not suitable for medical protective masks against SARS-CoV-2.

US 2008/295843 discloses a heat molded reusable facemask having at least a filter layer of material for trapping particles with a size range of known contaminant particles, and at least a porous layer comprising zeolite carriers embedded at the surface of fibers. A mask can be constructed from multiple layers, wherein two filter layers having different filtering specifications are combined with two sanitizing layers, which are more porous than the filter layers, and serve to sanitize contaminants passing there through prior to reaching filter layers; the porous layers comprising zeolite are the outermost, respectively the innermost layers. The material used in the filter elements of the mask, i.e zeolite carrier that contains silver and / or copper ions, has antimicrobial properties.

In addition, for the mask materials containing copper in the prior art, such as patent No CN 100490925C for "An Antibacterial And Antiviral Mask And Manufacturing Method", patent No. CN 102171322B for "A Dry Disinfection Patch Used To Reduce The Spread of Virions", and patent publication No. CN 111235871A for an "Antiviral Filter Layer Made of Copper-containing Chitosan Fiber And Its Application", patent publication No. CN111264932A for "A New Type of Antibacterial Surgical Protective Mask", etc., the copper element must inevitably destroy the cell structure and then make the cytotoxicity increase to achieve high antibacterial and antiviral performance.

In summary, the problem with the prior art is that materials containing copper (elemental copper, cuprous compound and cupric compound, including copper ion-modified zeolite materials) can be used for civilian masks, but they cannot be used for medical masks with high biological safety requirements. For example, ordinary medical masks or medical surgical masks must comply with the relevant regulations on cytotoxicity in the industry standards (YYT 0969-2013 "Disposable Medical Masks" and YY 0469-2011 "Medical Surgical Mask"), and the cytotoxicity must be implemented strictly in accordance with the national standard "Biological Evaluation of Medical Devices Part 5: In Vitro Cytotoxicity Test" GB/T 16886.5-2017/ISO 10993-5:2009. In addition, the cytotoxicity of copper is positively correlated with antibacterial and antiviral performance. In order to achieve good antibacterial and antiviral performance, the content of copper must be high enough, but the cytotoxicity will increase accordingly. That is, in order to achieve the cytotoxic biosafety index of medical masks or medical surgical masks, antibacterial and antiviral performance cannot be achieved; on the contrary, to achieve efficient antibacterial and antiviral performance, it is necessary to sacrifice the requirements of cytotoxicity and fail to meet the requirements for biosafety of medical masks. That is, the existing technology cannot meet the high enough antibacterial and antiviral performance of copper-containing materials and low enough cytotoxicity at the same time. Further, according to the structure of the existing mask cover structure, it is also impossible to realize the use of a copper-containing material mask for medical masks with high biological safety requirements.

### Summary of the Disclosure

In view of the shortcomings of the prior art, the first object of this disclosure is to provide a multilayer copper-based zeolite fiber medical material, which comprises copper, but at the same time has sufficiently low cytotoxicity and sufficiently high antibacterial and antiviral performance. It can be used as a medical material with high biological safety requirements, and especially suitable for being used as a biosafety medical material to kill SARS-CoV-2.

Based on the existing problems in the prior art, through many unremitting experimental studies, the inventors unexpectedly found that the medical material as provided can use the copper ions locked on the surface of the copper-based zeolite fiber layer to interact with biological macromolecules such as bacterial or virus proteins/amino acids and thus kill bacteria or viruses after the bacteria or virus attaches the mask. The medical material is obtained by the following technical means: laminating the copper-based zeolite fiber layer with a specific structure and the specific layer in a specific order; controlling the relative size of the pore size between the outermost copper-based zeolite fiber layer and the first intermediate layer; controlling the quality of the copper-based zeolite in gradient distribution from outside in along the radial interface. The prepared medical material of copper makes the copper-based zeolite fiber with cytotoxicity have high enough antibacterial and antiviral performance and low enough cytotoxicity under a specific macrostructure, which meets the regulations on cytotoxicity in the medical mask industry standard, breaks the traditional academic thinking, breaks through the technical obstacles of the existing technology, and overcomes the technical prejudice of the application of copper-containing materials in medical materials.

It should be particularly pointed out that the cytotoxicity described in the present disclosure is tested in strict accordance with the national standard "Medical Device Biological Evaluation Part 5: In Vitro Cytotoxicity Test" GB/T 16886.5-2017/ISO 10993-5:2009.

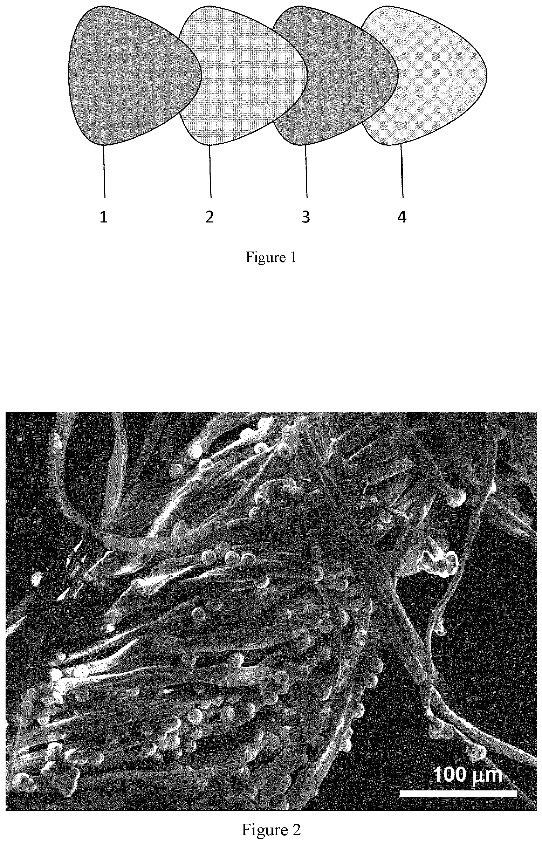

The present disclosure provides a multilayer copper-based zeolite fiber medical material according to claim 1, which includes a four-layer structure: the outermost layer, the first intermediate layer, the second intermediate layer, and the innermost layer in sequence; the outermost layer and second intermediate layer are copper-based zeolite fiber layers, the first intermediate layer is a hydrophobic fiber layer, and the innermost layer is a hydrophilic fiber layer; the pore size of the copper-based zeolite fiber layer of the outermost layer is greater than that of the hydrophobic fiber layer of the first intermediate layer; the copper-based zeolite fiber layer comprises copper-based zeolite and fiber threads, and the fiber threads are formed by twisting fibers; the copper-based zeolite is independently dispersed on the fiber surface, and the mass of copper-based zeolite decreases in a gradient from outside to inside along the radial interface of the fiber thread (as shown in Figures 1, 2, and 3).

The pore size refers to the maximum pore size distribution range of the pores in the fiber layer; further, the actual pore size of each pore refers to the diameter of the smallest particle that cannot pass through the pores of the fiber layer.

The innermost layer of the medical material refers to the side attached to the body of the wearer.

The outermost layer of the medical material refers to the side away from the wearer's body.

The copper-based zeolite means that the surface of the zeolite or the inside/outside of the zeolite framework comprises copper ions, and the copper ions are selected from the group consisting of Cu¹⁺ and Cu²⁺.

The copper ions in the zeolite framework refer to the framework structure of the zeolite composed of copper ions and Si, Al or O elements.

The copper ions outside the framework of the zeolite refer to the metal ions outside the framework of the copper-based zeolite to balance charge; the charge-balancing metal ion outside the copper-based zeolite framework refers to balance the anionic framework structure of the zeolite composed of [SiO₄]⁰ and [AlO₄]⁻ tetrahedron as a cation.

The hydrophilic fiber layer is composed of hydrophilic fibers; the hydrophilic fiber refers to the macromolecular chain of the fiber with a certain number of strong polar groups (such as -OH, -NH₂, -C= O etc.).

The hydrophobic fiber layer is composed of low-density and hydrophobic or water-resistant fiber; the hydrophobic fiber is selected from the group consisting of polyethylene, polypropylene, polyester, modified polyethylene, modified polypropylene, and modified polyester. Typically, a low-density point-bonded non-woven fabric layer made of polyethylene terephthalate fiber and polyethylene under pressure for point bonding processing can be used.

The mechanism that the medical material of the present disclosure has sufficiently high antibacterial/antiviral performance and sufficiently low cytotoxicity is as follows:

Since the hydrophobic fiber layer has a smaller pore size than the copper-based zeolite fiber layer and its surface has a water-retaining effect, which refers to the extremely small water droplets formed on the surface of the hydrophobic fiber layer (adjacent to the copper-based zeolite fiber layer), it is beneficial to limit the small amount of copper ions eluted from copper-based zeolite within the local area of the copper-based zeolite fiber layer. The mass of the copper-based zeolite decreases along the radial interface of the fiber thread from the outer circumference of the fiber thread to inside gradually (the greater the mass of the copper-based zeolite is, the greater the concentration of copper ions contained in it). Due to the diffusion effect of ions in the solution from high concentration to low concentration, when the water vapor exhaled during the wearing of the mask infiltrates the copper-based zeolite fiber layer, the copper ions locked in the zeolite pores on the surface of the fiber thread have a trend to move into the fiber thread. Since the internal framework of the zeolite is limited, the copper ions tend to move from the outer circumference of the fiber to the inside, they are still locked in the pores of the zeolite on the surface of the fiber. The above-mentioned copper ions are selected from the group consisting of Cu¹⁺ and Cu²⁺. The size of cells is generally 5-200 µm, while the size of bacteria is generally 0.2-5 µm, and the size of viruses is generally below 200 nm. As the copper ions are confined in the pores of the copper-based zeolite, and the small amount of copper ions eluted from copper-based zeolite are limited to the local area of the copper-based zeolite fiber layer, it is difficult to contact with cells, which reduces the possibility of interaction with the cells and greatly decreases cytotoxicity. Because of the small size of bacteria and viruses, they can be adsorbed by the copper-based zeolite of the copper-based zeolite fiber layer. At this time, the copper ions locked in the zeolite pores and a small amount of copper ions distributed in the local area of the fiber layer will act on the bacteria and viruses, so as to play an excellent performance in killing bacteria and viruses.

Preferably, the copper-based zeolite comprises copper element having electron acceptor.

The electron acceptor means that the copper element in the copper-based zeolite has the ability to accept electrons from the surface groups of the copper-based zeolite.

Preferably, the charge-balancing metal ion outside the copper-based zeolite framework comprises Cu²⁺.

Preferably, the copper-based zeolite comprises a group containing a lone electron pair and/or a group containing a conjugation effect.

The group containing a lone pair of electrons means that the outermost electron layer of the group has at least one atom with non-bonding electrons that are not used to form a covalent bond.

The group containing conjugation effect means that the group comprises π electrons, which can change the electron cloud distribution in the conjugated system.

The copper-based zeolite comprises a -COOH group and/or a -COO⁻ group.

Preferably, the copper-based zeolite includes a N group containing a lone pair of electrons; preferably, the N group containing a lone pair of electrons includes a -NH₂ group.

Preferably, a melt-blown non-woven fabric layer can be added between the first intermediate layer and the second intermediate layer.

The composition of the melt-blown non-woven fabric includes any one or more of polyethylene, polypropylene, polyester, modified polyethylene, modified polypropylene, and modified polyester.

Preferably, the pore size of the copper-based zeolite fiber layer of the second intermediate layer is greater than the pore size of the innermost hydrophilic fiber layer.

Preferably, the particle size D90 of the copper-based zeolite is 0.5 to 20 µm; preferably, the particle size D90 of the copper-based zeolite is 1 to 15 µm; more preferably, the particle size D90 of the copper-based zeolite is 5 to 10 µm.

D90 refers to the particle size when the cumulative particle size distribution percentage of the microscopic particles of the copper-based zeolite on the fiber surface reaches 90%. Its physical meaning is that the microscopic particles of copper-based zeolite with a particle size larger than it account for 10%, and the microscopic particles of copper-based zeolite smaller than it account for 90%. The microscopic particles of the copper-based zeolite are zeolite geometric bodies with a certain shape that retain the boundary formed by the growth of the original copper-based zeolite and a size of less than 50 µm.

Preferably, the mass fraction of copper in the copper-based zeolite fiber layer is 0.02 to 4 wt%; preferably, the mass fraction of copper in the copper-based zeolite fiber layer is 0.5 to 2 wt%; more preferably, the mass fraction of copper in the copper-based zeolite fiber layer is 1 to 1.5 wt%.

Preferably, the Cu²⁺ accounts for 5% to 40% of the charge-balanced metal ion content outside the framework of the copper-based zeolite; preferably, the Cu²⁺ accounts for 10% to 30% of the charge-balanced metal ion content outside the framework of the copper-based zeolite; Preferably, the Cu²⁺ accounts for 15% to 20% of the charge balance metal ion content outside the framework of the copper-based zeolite.

The metal ion content refers to the proportion of metal ion atoms.

Preferably, the Si/Al ratio of the copper-based zeolite is 1.5 to 5; preferably, the Si/Al ratio of the copper-based zeolite is 1.8 to 3; preferably, the Si/Al ratio of the copper-based zeolite is 2 to 2.5.

The Si/Al ratio of the copper-based zeolite is the ratio of the number of atoms of the two.

Preferably, the copper-based zeolite independently dispersed on the fiber surface has its own independent boundary.

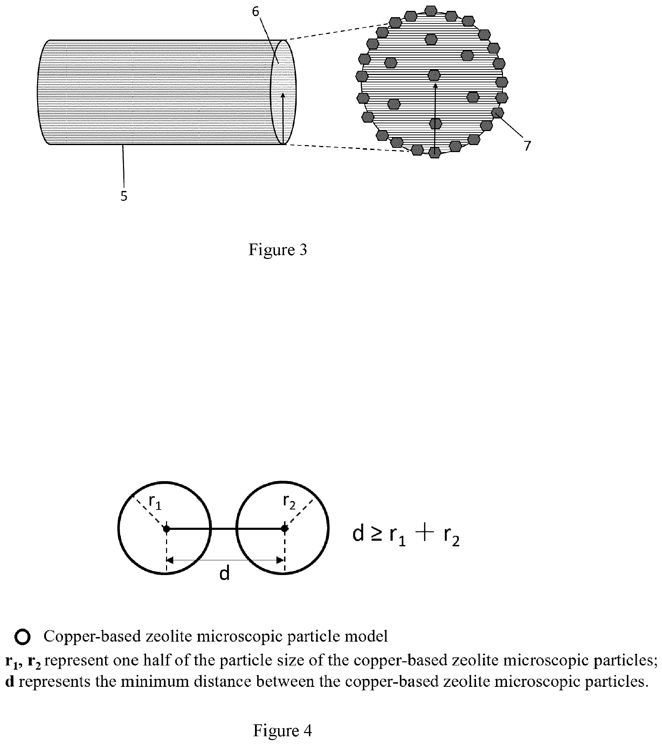

The independent dispersion means that the minimum distance between the copper-based zeolite microscopic particles and the nearest copper-based zeolite microscopic particles is greater than or equal to one-half of the sum of the particle diameters of the two copper-based zeolite microscopic particles, that is: d ≥ r1+r2; wherein r1 and r2 respectively represent one-half of the diameter of two adjacent copper-based zeolite microscopic particles; d represents the minimum distance between two adjacent copper-based zeolite microscopic particles (as shown in the Figure 4).

Preferably, the copper-based zeolite is composed of zeolite nanoparticles.

Preferably, the copper-based zeolite is selected from the group consisting of X-type zeolite, Y-type zeolite, A-type zeolite, chabazite, L-type zeolite, and P-type zeolite.

Preferably, the fibers of the copper-based zeolite fiber layer are selected from the group consisting of rayon fibers, acetate fibers, carboxymethyl cellulose, bamboo fibers, cotton fibers, wood fibers, polypropylene fibers, and polyethylene fibers.

The second object of the present disclosure is to provide a method for manufacturing a multilayer copper-based zeolite fiber medical material according to claim 10, which includes the following steps:
1) manufacturing a hydrophobic fiber layer and a hydrophilic fiber layer;
2) manufacturing a copper-based zeolite fiber layer, and the manufacturing method of the copper-based zeolite fiber layer includes:
   (a) twisting the fibers to form a fiber thread, and synthesizing a fiber thread containing copper-based zeolite by using the fiber thread as a scaffold for zeolite nucleation and growth, wherein the content of copper-based zeolite decreases along the radial interface of the fiber thread from the outside to the inside gradient;
   (b) providing a copper-based zeolite fiber layer by weaving the copper-based zeolite-containing fiber thread as warp and weft;
3) preparing a four-layer antibacterial and antiviral medical material in the order of a copper-based zeolite fiber layer, a hydrophobic fiber layer, a copper-based zeolite fiber layer, and a hydrophilic fiber layer; wherein the outermost copper-based zeolite fiber layer has a pore size greater than that of the hydrophobic fiber layer.

Preferably, a melt-blown non-woven fabric layer can be added between the hydrophobic fiber layer and the hydrophilic fiber layer.

The hydrophilic fiber layer is composed of hydrophilic fibers; the hydrophilic fiber refers to the macromolecular chain of the fiber with a certain number of strong polar groups (such as -OH, -NH₂, -C=O, etc.).

The hydrophobic fiber layer is composed of fibers with low density and strong hydrophobicity or water resistance; the hydrophobic fibers are selected from the group consisting of polyethylene, polypropylene, polyester, modified polyethylene, modified polypropylene, and modified polypropylene. Typically, a low-density point-bonded non-woven fabric layer made of polyethylene terephthalate fiber and polyethylene for point-bonding processing can be used.

Further disclosed is a medical mask which comprises any one of the aforementioned multi-layer copper-based zeolite fiber medical materials or the multi-layer copper-based zeolite fiber medical material prepared by any of the aforementioned manufacturing methods.

The innermost layer of the mask refers to the side close to the wearer's face.

The outermost layer of the mask refers to the side furthest away from the wearer's face.

The mask includes a cover body and ear straps connected on both sides of the cover.

The mask can be a medical mask or a civilian mask. The medical masks can be ordinary medical masks, medical surgical masks, and medical protective masks.

Further disclosed is a protective mask which comprises any one of the aforementioned multi-layer copper-based zeolite fiber medical materials or the multi-layer copper-based zeolite fiber medical material prepared by any of the aforementioned manufacturing methods.

The third object of the present disclosure is to provide a protective equipment which comprises any one of the aforementioned multi-layer copper-based zeolite fiber medical materials or the multi-layer copper-based zeolite fiber medical material prepared by any of the aforementioned manufacturing methods.

Further, the protective equipment refers to wearable equipment used for safety protection from virus or bacteria, and the wearables are selected from the group consisting of masks, protective masks, protective clothing, face screens, gloves, shoes, boots, caps, and earmuffs.

Compared with the prior art, the beneficial effects of the present disclosure are:
1. The present disclosure solves confrontational problem of the safety (cytotoxicity) and effectiveness (antibacterial and antiviral) of medical materials based on the copper-based zeolite fiber layer for the first time, and overcomes the technical prejudice that the existing technology cannot use copper-containing materials to make medical materials. The medical material provided by the present disclosure comprises four layers, which are laminated in the order of a copper-based zeolite fiber layer, a hydrophobic fiber layer, a copper-based zeolite fiber layer, and a hydrophilic fiber layer. The copper-based zeolite fiber layer includes copper-based zeolite and fiber threads. The fiber threads are formed by twisting fibers. The copper-based zeolite is independently dispersed on the fiber surface, and the content of the copper-based zeolite decreases gradually along the radial interface of the fiber thread from the outside to the inside. Through the overall design and control of the copper-based zeolite fiber layer-based macrostructure (four-layer fiber composite structure) and the copper-based zeolite fiber layer microstructure, the microstructure and macrostructure of medical materials play a synergistic effect on the whole, so as to produce medical materials with sufficiently low cytotoxicity and sufficiently efficient antibacterial and antiviral performance to meet the cytotoxicity regulations in the medical mask industry standard. This technical disclosure ensures that medical personnel and epidemic prevention personnel have very effective protective measures when facing confirmed patients, high-risk and susceptible environments, and environments with high numbers of bacteria and viruses, thereby greatly reducing the possibility of bacterial and virus infection.
2. Further, the present disclosure not only controls the release of copper ions through zeolite, but also introduces groups containing lone electron pairs and/or groups containing conjugation effect on the surface of copper-based zeolite, which allows on the one hand to further reduce the cytotoxicity of medical materials, and to improve the efficiency of killing bacteria and viruses through the action of the copper ions locked on the surface of the copper-based zeolite; on the other hand to further limit the dissolution of copper ions in water vapor and eliminate the safety hazards of long-term wear by medical staff.
3. Further, the melt-blown cloth can not only improve the filtering effect of medical materials, but also has the effect of electrostatic charge, which can further regulate and lock the active range of copper ions, and has a certain balance effect on the high antibacterial and antiviral performance and sufficiently low cytotoxicity of the present disclosure.
4. The present disclosure provides for the first time a medical material with dual inactivation of bacteria and viruses. The medical material provided by the present disclosure includes a four-layer structure, two of which are copper-based zeolite fiber layers. When droplets carrying bacteria and viruses are attached to the surface of medical materials, the outermost copper-based zeolite fiber layer can effectively kill bacteria and viruses. Therefore, on the one hand, the design of the present disclosure can not only prevent bacteria and viruses from entering the body; on the other hand, it can completely kill bacteria and viruses attached to the outer layer of medical materials, which prevents contaminated medical materials from becoming cross-infected and eliminates the risk of re-spreading bacteria and viruses. The second intermediate layer of the present disclosure is a copper-based zeolite fiber layer, and the innermost layer is a hydrophilic fiber layer. If the person wearing medical materials carries the virus source and exhales the saliva carrying the virus and bacteria, the saliva passes through the hydrophilic fiber layer to the copper-based zeolite, where the virus and bacteria in the saliva will be efficiently inactivated, thus can prevent the person carrying the virus source from spreading the virus and bacteria to the outside. In general, the multilayer copper-based zeolite fiber medical material of the present disclosure can double inactivate viruses, and has high-efficiency protection for both healthy people and patients.
5. The medical material of the present disclosure and the prepared protective equipment have two major technical advantages of low cytotoxicity and high antibacterial and viral performance, and there is no need to sacrifice cytotoxicity to ensure high killing performance of bacteria and viruses. The technology of the present disclosure has high efficiency in killing viruses (especially SARS-CoV-2 with high survivability and rapid mutation) and sufficient biological safety. It is expected to reshape the industry standard of medical masks, break the traditional academic thinking style, break through the technical defects of traditional medical protection in practice, and have high theoretical and practical value.

### Brief Description of the Drawings

Figure 1 is a schematic diagram of the arrangement of the layers of the multilayer copper-based zeolite fiber medical material of the present disclosure; 1-copper-based zeolite fiber layer, 2-hydrophobic fiber layer, 3-copper-based zeolite fiber layer, 4-hydrophilic fiber layer;
Figure 2 is a scanning electron micrograph of fiber threads containing copper-based zeolite in the copper-based zeolite fiber layer of the present disclosure;
Figure 3 is a schematic diagram showing the distribution of the copper-based zeolite of the present disclosure along the radial interface of the fiber thread; 5-fiber thread; 6-fiber thread radial interface; 7-copper-based zeolite; the radial interface of the fiber thread refers to the cross-section of the fiber thread; the mass of the copper-based zeolite decreases in a gradient from outside to the inside along the radial interface of the fiber thread (the mass of the copper-based zeolite shown in the figure decreases in the direction of the arrow);
Figure 4 is a schematic diagram of the positional relationship between the copper-based zeolite microparticles on the fiber surface of the copper-based zeolite fiber layer of the present disclosure, and the copper-based zeolite microparticles are independently dispersed on the fiber surface.

### Detailed Description of the Disclosure

The terms "comprising", "including", "having", "containing" or any other variations as used herein are intended to cover non-exclusive inclusion. For example, a composition, step, method, product, or device containing the listed elements is not necessarily limited to those elements, but may include other elements not explicitly listed or inherent elements in the composition, step, method, product, or device.

In addition, the indefinite articles "a" and "an" before an element or component of the present disclosure have no limitation on the quantity requirement (that is, the number of occurrences) of the element or component. Therefore, "a" or "an" should be interpreted as including one or at least one, and a singular form of an element or component also includes a plural form, unless the number clearly refers to the singular form.

Determination of the amount of copper ions dissolved: the medical material is immersed in MEM culture solution at a ratio of 0.1 g/mL for 24 hours to obtain an extract of the medical material. The obtained extract is detected with Inductively Coupled Plasma Emission Spectrometer to obtain Cu²⁺ concentration in the extract.

Cytotoxicity test (refer to the national standard "Biological Evaluation of Medical Devices Part 5: In Vitro Cytotoxicity Test" GB/T 16886.5-2017/ISO 10993-5:2009): After immersing the medical material in MEM culture solution at a ratio of 0.1 g/mL for 24 hours, an extract of the medical material is obtained. Place the Vero cell culture medium (1×10⁵) in a CO₂ incubator for 22 to 26 h. The culture medium is replaced by the extract of medical materials, and incubated for another 24 hours. Observe the morphological changes of Vero cells with a microscope. Then remove the medium and add 50 µL of MTT solution, incubate in a CO₂ incubator for 2 hours. The MTT solution is removed and 100 µL of isopropanol is added to each well. Shake the titer plate, and measure the absorbance at 570 nm. According to the measured absorbance, it is converted into the survival rate of the cell.

Material antibacterial performance test method: Preparation of a suspension of Gram-negative Escherichia coli (ATCC25922), Gram-negative Klebsiella pneumoniae (ATCC70063), Gram-positive Staphylococcus aureus (ATCC6538). a). Dilute the nutrient broth 500 times with distilled water and use it as a diluent after autoclaving. After the inoculation loop is flame-sterilized, take a loop of slant nutrient agar medium in a test tube containing the dilution solution, and use a 10-fold dilution method to dilute the bacteria solution to an appropriate concentration [about (1 - 9)×10⁵CFU/ mL], which is the experimental bacterial solution, b) Spread the medical material to be tested in a sterilized petri dish, draw 0.15 mL of the bacterial suspension drop on the medical material, and use the 400 mm±2 mm square covering film to make the bacterial suspension evenly cover the medical materials, but not beyond the edge of the film. Cover the petri dish and place it at room temperature for 1 h to count the viable bacteria. c) Live bacteria count. Rinse the medical materials and coverings repeatedly with 10 mL of SCDLP broth. Dilute 1 mL washing solution 10 times with 9 mL phosphate buffer solution and mix well, pipette 1 mL into a petri dish. Add 15 mL-20 mL agar (46°C-48°C) to mix, and put it in a biochemical incubator (36±1 °C) and cultivate in for 40 h to 48 h after solidification. Count the number of viable bacteria in the plate according to the method of GB4889.2, and calculate the sterilization rate.

The test method for the inactivation of SARS-CoV-2: Place the medical material in a 6-well plate with a concave bowl in the middle, and slowly drop 0.5 mL of the virus onto the medical material (30 s) to ensure the virus liquid is absorbed by the mask and will not drip. Incubate for a certain period of time (15 minutes). Add 2 mL of culture medium to each well, fully infiltrate for 2 minutes, and then discard the mask. 1 mL of Vero cell culture medium (5×10⁵) is added to each well and mixed, then placed in a CO₂ incubator for 72 h. Observe cell pathology and detect viral nucleic acid (PCR). Aspirate 200 µL of the cell culture supernatant, and extract the viral nucleic acid with a magnetic bead method nucleic acid extraction kit (MVR01) and an automatic nucleic acid extractor. The final elution volume is 50 µL. Take 5 µL of nucleic acid extract, use a one-step novel coronavirus nucleic acid detection kit to detect the level of viral nucleic acid, and calculate the material's inhibition rate against the virus based on the Ct value.

The embodiment of the present disclosure uses a mask as an example to illustrate the manufacturing method, structural characteristics, biological safety (such as cytotoxicity), and antibacterial and antiviral performance of the multilayer copper-based zeolite fiber medical material and the prepared mask.

### Example 1

The manufacturing method of the mask 1 of the present disclosure includes the following steps:
The mask includes the outermost layer, the first intermediate layer, the second intermediate layer and the innermost layer; the outermost layer and the second intermediate layer are composed of copper-based zeolite fiber layers; the first intermediate layer is a hydrophobic fiber layer; the innermost layer is a hydrophilic fiber layer.

Among them, the first intermediate layer is composed of low-density and hydrophobic or water-resistant fibers. Typically, a low-density point-bonded non-woven fabric layer made of polyethylene terephthalate fiber and polyethylene subjected to pressure point bonding processing can be used. The pore size of the first intermediate layer is set at 40-60µm.

The innermost layer is a layer of water-absorbing non-woven fabric.

Among them, the manufacturing methods of the copper-based zeolite fiber layer of the outermost layer and the second intermediate layer are as follows:
(1) Preparing the zeolite precursor solution according to the following molar ratio of 8Na₂O:Al₂O₃:9SiO₂:180H₂O. 60 cotton fiber were twisted and spun into fiber threads. The zeolite precursor solution and the fiber threads were mixed uniformly and heat treated at 110 °C for 36 h to obtain Y-type zeolite fiber threads. After soaking in a 5 M copper sulfate solution for 3 times, each time for 3 h, rinsing with deionized water several times, fiber threads containing copper-based zeolite are obtained.
(2) Knitting up a copper-based zeolite fiber layer by using the copper-based zeolite-containing fiber thread as warp and weft. The pore size of the copper-based zeolite fiber layer was set to 100 µm.

The Si/Al ratio of the copper-based zeolite is 2; Cu²⁺ accounts for 20% of the charge-balanced metal ion content outside the framework of the copper-based zeolite; and the mass fraction of copper in the fiber layer of the copper-based zeolite is 2 wt%.

Through observation of the prepared copper-based zeolite fiber thread by scanning electron microscope, the fiber thread is formed by spirally winding fibers, and the copper-based zeolite with an average particle size of 7 µm hemispherical is independently dispersed on the surface of the fiber, and the content of copper-based zeolite decreases gradually along the radial interface of the fiber thread from the outside to the inside. Observing the obtained copper-based zeolite through a scanning electron microscope, the copper-based zeolite is a microscopic particle composed of nanoparticles, which makes the copper-based zeolite and the fiber tightly combined. Among them, the pore size of the copper-based zeolite fiber layer of the outermost layer is greater than that of the hydrophobic fiber layer of the first intermediate layer; the pore size of the copper-based zeolite fiber layer of the second intermediate layer is greater than that of the innermost fiber layer.

The copper-based zeolite fiber layer, hydrophobic fiber layer, copper-based zeolite fiber layer, and hydrophilic fiber layer are sequentially heat-sealed to prepare the cover of the mask 1 of the present disclosure.

### Comparative Example 1

The copper-based zeolite fiber layer prepared in Example 1 was used as Comparative Example 1, and was compared with Example 1.

### Comparative Example 2

The hydrophobic fiber layer, copper-based zeolite fiber layer, copper-based zeolite fiber layer, and hydrophilic fiber layer are sequentially heat-sealed to prepare the cover 1 of the comparative mask, which was compared with Example 1.

### Comparative Example 3

The copper-based zeolite fiber layer, the copper-based zeolite fiber layer, and the hydrophilic fiber layer are sequentially heat-sealed to prepare the cover 2 of the comparative mask, which was compared with Example 1.

### Comparative Example 4

The copper-based zeolite fiber layer, the hydrophobic fiber layer, and the copper-based zeolite fiber layer was sequentially heat-sealed to prepare the cover 3 of the comparative mask, which was compared with Example 1.

### Comparative Example 5

Refer to the Chinese publication No. 111469498A for the manufacturing method of the medical protective material containing copper ion antibacterial fabric.

The "antibacterial fabric layer" of the primary filter layer, the antibacterial fabric layer, the precision filter layer, and the skin-friendly comfort layer arranged in sequence from outside in is replaced with the "copper-based zeolite fiber layer" described in the present disclosure, and reintegrated into a medical protective material of Comparative Example 5

According to the above method of the present disclosure, the medical protective materials prepared in Example 1 and Comparative Examples 1-5 were tested for copper ion elution, antibacterial, antiviral, and cytotoxicity. The results are shown in Table 1.

**Table 1 Comparison of cytotoxicity of mask materials.**

| Number | Material | Copper ion elution/ppm | Cell survival rate/% | Antibacterial rate/% (*Escherichia coli*) | Antiviral rate/% (SARS-CoV-2) |
|---|---|---|---|---|---|
| Example 1 | Cover of the mask 1 in the present disclosure | 1 | 80 | 99.97 | 99.95 |
| Comparative Example 1 | Copper-based zeolite fiber layer | 80 | 0.5 | 99.99 | 99.99 |
| Comparative Example 2 | Cover 1 of comparative mask | 8 | 45 | 56.67 | 60.98 |
| Comparative Example 3 | Cover 2 of comparative mask | 40 | 1.0 | 99.95 | 99.99 |
| Comparative Example 4 | Cover 3 of comparative mask | 10 | 20 | 89.78 | 93.99 |
| Comparative Example 5 | Medical protective material of comparative Example 5 | 20 | 5 | 90.68 | 95.64 |

Comparing Example 1 with Comparative Examples 1-4 (Table 1), the copper-based zeolite fiber layer has strong cytotoxicity; however, the mask cover composed of copper-based zeolite fiber layer, hydrophobic fiber layer, copper-based zeolite fiber layer and hydrophilic fiber layer stacked in sequence has less cytotoxicity and meets the cytotoxicity regulations in the medical mask industry standard. In addition, if the hydrophobic fiber layer and hydrophilic fiber layer of the present disclosure are reduced, or the relative position of the hydrophobic fiber layer is changed (Comparative Example 1), the amount of copper ions leached out will increase, and the cytotoxicity of the mask body will increase, which does not meet the biosafety standards of medical masks. By laminating the copper-based zeolite fiber layer and the specific mask layer of the present disclosure in a specific order, controlling the relative pore size of the outermost copper-based zeolite fiber layer and the first intermediate layer and the quality of the copper-based zeolite along the radial interface gradient distribution from the outside to the inside, locking water by the surface of the hydrophobic fiber layer and the electrostatic adsorption of copper ions and other synergistic effects, the amount of copper ions eluted is limited and the possibility of interaction with cells is reduced, thus the cytotoxicity is greatly reduced; bacterial and viruses are adsorbed by the zeolite, and then the activity of the bacterial and virus is inhibited by the high concentration of copper ions on the surface of the copper zeolite, which has an effective anti-bacterial and anti-virus effect.

It can be obtained from Comparative Examples 1-4 that the amount of copper ions dissolved out is related to cytotoxicity, antibacterial and antiviral rate. The low content of copper element cannot achieve good antibacterial and antiviral performance effects (<70%); on the contrary, in order to achieve high antibacterial and antiviral performance (>90%), the high content of copper element is required, which is unable to meet the cytotoxicity regulations in the medical mask industry standard.

From the comparison of the medical protective materials of Example 1 and Comparative Example 5, even if the prior art replaces the antibacterial and antiviral functional layer in the medical protective material with the copper-based zeolite fiber layer of the present disclosure, it still cannot achieve low cells toxicity and high antibacterial and antiviral performance at the same time. This shows that the overall design and control of macrostructure and microstructure of the copper-based zeolite fiber layer unexpectedly discovered by the present disclosure make the microstructure and macrostructure of the mask play a synergistic effect on the whole, making it have high antibacterial and antiviral performance and low cytotoxicity.

### Comparative Example 6

The difference from Example 1 is that the content of the copper-based zeolite is equal from outside to inside along the radial interface of the fiber thread in the prepared copper-based zeolite fiber layer. And the rest of the manufacturing steps are the same. The cover 4 of the comparative mask is obtained.

Among them, the manufacturing methods of the copper-based zeolite fiber layer of the outermost layer and the second intermediate layer are as follows:
(1) Preparing the zeolite precursor solution according to the following molar ratio of 8Na₂O:Al₂O₃:9SiO₂:180H₂O. The cotton fiber and the zeolite precursor solution were mixed uniformly and heat-treated at 110 °C for 36 h to obtain Y-type zeolite fiber. After soaking in a 5M copper sulfate solution for 3 times, each time for 3 h, rinsing with deionized water several times, fiber threads containing copper-based zeolite are obtained.
   60 fibers containing copper-based zeolite are twist-spun into fiber threads containing copper-based zeolite.
(2) The copper-based zeolite-containing fiber threads are used as warp and weft to weave a copper-based zeolite fiber layer; the content of the copper-based zeolite is equal everywhere along the radial interface of the fiber thread from outside in.

### Comparative Example 7

The difference from Example 1 is that the content of the copper-based zeolite increases gradually along the radial interface of the fiber line from outside to inside in the prepared copper-based zeolite fiber layer. The rest of the manufacturing steps are the same. The cover 5 of the comparative mask is obtained.

Among them, the manufacturing methods of the copper-based zeolite fiber layer of the outermost layer and the second intermediate layer are as follows:
(1) Preparing the zeolite precursor solution according to the following molar ratio of 8Na₂O:Al₂O₃:9SiO₂:180H₂O. 60 cotton fibers were twisted and spun into fiber threads. The zeolite precursor solution and the fiber threads were mixed uniformly and heat treated at 110°C for 36 h to obtain Y-type zeolite fiber threads. After soaking in 5 M copper sulfate solution for 3 times, each time for 3 h, rinsing with deionized water several times, fiber threads containing copper-based zeolite are obtained (before).
(2) Untying the fiber thread containing copper-based zeolite (before), rearrange the fiber containing copper-based zeolite, so that the content of copper-based zeolite gradually increases from the outside to the inside. 60 copper-based zeolite-containing fibers are re-twisted into fiber threads containing copper-based zeolite.

The fiber thread containing the copper-based zeolite obtained above is used as warp and weft to knit into a copper-based zeolite fiber layer; the content of the copper-based zeolite increases gradually along the radial interface of the fiber thread from outside to inside.

**Table 2 Comparison of cytotoxicity and antiviral performance of mask materials**

| Number | Material | Copper ion elution/ppm | Cell survival rate/% | Antiviral rate/% (SARS-CoV-2) |
|---|---|---|---|---|
| Example 1 | Cover of the mask 1 in this disclosure | 1 | 80 | 99.95 |
| Comparative Example 6 | Cover 4 of comparative mask | 9 | 20 | 95.35 |
| Comparative Example 7 | Cover 5 of comparative mask | 8 | 45 | 30.45 |

From the comparison of Example 1 with Comparative Examples 6 and 7 (Table 2), the content of the copper-based zeolite of the present disclosure decreases gradually from the outside to the inside along the radial interface of the fiber thread, which plays a coordinated role in balancing the cytotoxicity and efficiency of inactivating SARS-CoV-2, so that the overall mask material has low cytotoxicity (high cell survival rate) and high virus inactivation. In contrast, for the other two types of copper-based zeolite distribution in the fiber thread, either high cytotoxicity and high virus inactivation, or slightly lower cytotoxicity and low virus inactivation, low cytotoxicity and high inactivation of the virus cannot be achieved at the same time, which cannot meet the cytotoxicity regulations in the medical mask industry standard.

### Example 2

The manufacturing method of the mask 2 of the present disclosure includes the following steps:
The mask includes the outermost layer, the first intermediate layer, the second intermediate layer and the innermost layer; the outermost layer and the second intermediate layer are composed of copper-based zeolite fiber layers; the first intermediate layer is a hydrophobic fiber layer; the innermost layer is a hydrophilic fiber layer.

Among them, the manufacturing methods of the copper-based zeolite fiber layer of the outermost layer and the second intermediate layer are as follows:
(1) Preparing the zeolite precursor solution according to the following molar ratio of 8Na₂O:Al₂O₃:9SiO₂:180H₂O. 60 cotton fiber were twisted and spun into fiber threads. The zeolite precursor solution and the fiber threads were mixed uniformly and heat treated at 110°C for 36 h to obtain Y-type zeolite fiber threads. After soaking in a 5M copper sulfate solution for 3 times, each time for 3 h, rinsing with deionized water several times, fiber threads containing copper-based zeolite are obtained.
(2) The copper-based zeolite-containing fiber threads are used as warp and weft to knit into a copper-based zeolite fiber layer.
(3)Surface modification of copper-based zeolite with -COO- groups.

The Si/Al ratio of the copper-based zeolite is 2; Cu²⁺ accounts for 20% of the charge-balanced metal ion content outside the framework of the copper-based zeolite; the mass fraction of copper in the fiber layer of the copper-based zeolite is 2 wt%.

Through observation of the prepared copper-based zeolite fiber thread by scanning electron microscope, the fiber thread is formed by spirally winding fibers, and the copper-based zeolite with an average particle size of 7 µm hemispherical is independently dispersed on the surface of the fiber, and the content of copper-based zeolite decreases gradually along the radial interface of the fiber line from the outside to the inside. Observing the obtained copper-based zeolite through a scanning electron microscope, the copper-based zeolite is a microscopic particle composed of nanoparticles, which makes the copper-based zeolite and the fiber tightly combined. Among them, the pore size of the copper-based zeolite fiber layer of the outermost layer is greater than that of the hydrophobic fiber layer of the first intermediate layer; the pore size of the copper-based zeolite fiber layer of the second intermediate layer is greater than that of the innermost fiber layer.

The above-mentioned copper-based zeoite fiber layer, hydrophobic fiber layer, copper-based zeolite fiber layer, and hydrophilic fiber layer are sequentially heat-sealed to prepare the cover of the mask 2 of the present disclosure.

The copper-based zeolite fiber layer modified with -COO⁻ groups, a hydrophobic fiber layer, a copper-based zeolite fiber layer modified with -COO⁻ groups, and a hydrophilic fiber layer are prepared by heat sealing to obtain the cover of mask 2 of the present disclosure.

### Example 3

The manufacturing method of the mask 3 of the present disclosure includes the following steps:

The mask includes the outermost layer, the first intermediate layer, the second intermediate layer and the innermost layer; the outermost layer and the second intermediate layer are composed of copper-based zeolite fiber layers; the first intermediate layer is a hydrophobic fiber layer; the innermost layer is a hydrophilic fiber layer.

Among them, the manufacturing methods of the copper-based zeolite fiber layer of the outermost layer and the second intermediate layer are as follows:
(1) Preparing the zeolite precursor solution according to the following molar ratio of 8Na₂O:Al₂O₃:9SiO₂:180H₂O. 60 cotton fiber were twisted and spun into fiber threads. The zeolite precursor solution and the fiber threads were mixed uniformly and heat treated at 110°C for 36 h to obtain Y-type zeolite fiber threads. After soaking in a 5 M copper sulfate solution for 3 times, each time for 3 h, rinsing with deionized water several times, fiber threads containing copper-based zeolite are obtained.
(2) The copper-based zeolite-containing fiber threads are used as warp and weft to knit into a copper-based zeolite fiber layer.
(3) Surface modification of copper-based zeolite with -NH₂ groups.

The Si/Al ratio of the copper-based zeolite is 2; Cu²⁺ accounts for 20% of the charge-balanced metal ion content outside the framework of the copper-based zeolite; the mass fraction of copper in the fiber layer of the copper-based zeolite is 2 wt%.

Through observation of the prepared copper-based zeolite fiber thread by scanning electron microscope, the fiber thread is formed by spirally winding fibers, and the copper-based zeolite with an average particle size of 7 µm hemispherical is independently dispersed on the surface of the fiber, and the content of copper-based zeolite decreases gradually along the radial interface of the fiber line from the outside to the inside. Observing the obtained copper-based zeolite through a scanning electron microscope, the copper-based zeolite is a microscopic particle composed of nanoparticles, which makes the copper-based zeolite and the fiber tightly combined. Among them, the pore size of the copper-based zeolite fiber layer of the outermost layer is greater than that of the hydrophobic fiber layer of the first intermediate layer; the pore size of the copper-based zeolite fiber layer of the second intermediate layer is greater than that of the innermost fiber layer.

The above-mentioned copper-based zeolite fiber layer, hydrophobic fiber layer, copper-based zeolite fiber layer, and hydrophilic fiber layer are sequentially heat-sealed to prepare the cover body of the mask 3 of the present disclosure.

The copper-based zeolite fiber layer modified with -NH₂ groups, the hydrophobic fiber layer, the copper-based zeolite fiber layer modified with -NH₂ groups, and the hydrophilic fiber layer are sequentially heat-sealed to prepare the cover of the mask 3 of the present disclosure.

### Example 4

The manufacturing method of the mask 4 of the present disclosure includes the following steps:
The mask includes the outermost layer, the first intermediate layer, the second intermediate layer and the innermost layer; the outermost layer and the second intermediate layer are composed of copper-based zeolite fiber layers; the first intermediate layer is a hydrophobic fiber layer; the innermost layer is a hydrophilic fiber layer.

Among them, the manufacturing methods of the copper-based zeolite fiber layer of the outermost layer and the second intermediate layer are as follows:

Preparing the zeolite precursor solution according to the following molar ratio of 8Na₂O:Al₂O₃:9SiO₂:180H₂O. 60 cotton fiber were twisted and spun into fiber threads. The zeolite precursor solution and the fiber threads were mixed uniformly and heat treated at 110°C for 36 h to obtain Y-type zeolite fiber threads. After soaking in a 5 M copper sulfate solution for 3 times, each time for 3 h, rinsing with deionized water several times, fiber threads containing copper-based zeolite are obtained.

The copper-based zeolite-containing fiber threads are used as warp and weft to knit into a copper-based zeolite fiber layer.

Surface modification of copper-based zeolite with -NH₂ group and -COO⁻ group at the same time.

The Si/Al ratio of the copper-based zeolite is 2; Cu²⁺ accounts for 20% of the charge-balanced metal ion content outside the framework of the copper-based zeolite; and the mass fraction of copper in the fiber layer of the copper-based zeolite is 2 wt%.

Through observation of the prepared copper-based zeolite fiber thread by scanning electron microscope, the fiber thread is formed by spirally winding fibers, and the copper-based zeolite with an average particle size of 7 µm hemispherical is independently dispersed on the surface of the fiber, and the content of copper-based zeolite decreases gradually along the radial interface of the fiber line from the outside to the inside. Observing the obtained copper-based zeolite through a scanning electron microscope, the copper-based zeolite is a microscopic particle composed of nanoparticles, which makes the copper-based zeolite and the fiber tightly combined. Among them, the pore size of the copper-based zeolite fiber layer of the outermost layer is greater than that of the hydrophobic fiber layer of the first intermediate layer; the pore size of the copper-based zeolite fiber layer of the second intermediate layer is greater than that of the innermost fiber layer.

The copper-based zeolite fiber layer, hydrophobic fiber layer, copper-based zeolite fiber layer, and hydrophilic fiber layer are sequentially heat-sealed to prepare the cover body of the mask 4 of the present disclosure.

The copper-based zeolite fiber layer modified with -NH₂, -COO⁻ groups, hydrophobic fiber layer, copper-based zeolite fiber layer modified with -NH₂, -COO⁻ groups, and hydrophilic fiber layer are sequentially heat-sealed to prepare the cover of the mask 4 of the present disclosure.

**Table 3 Comparison of cytotoxicity and antiviral, antibacterial performance of mask materials**

| Number | Copper ion elution/ppm | Cell survival rate/% | Antibacterial rate (*E*. *coli*)/% | Antibacterial rate (*Pneumoniae*)/*%* | Antibacterial rate (*Staphylococcus aureus*)/*%* | Antiviral rate (SARS-CoV-2)/% |
|---|---|---|---|---|---|---|
| Example 1 | 1 | 80 | >99.99 | >99.99 | >99.99 | 99.95 |
| Example 2 | 0.5 | 92 | >99.99 | >99.99 | >99.99 | 99.99 |
| Example 3 | 0.5 | 93 | >99.99 | >99.99 | >99.99 | 99.98 |
| Example 4 | 0.05 | 97 | >99.99 | >99.99 | >99.99 | 99.999 |

It can be obtained from Examples 1, 2, 3, and 4 (Table 3) that although the copper-based zeolite fiber layer has strong cytotoxicity, the mask sequentially stacked by copper-based zeolite fiber layer, hydrophobic fiber layer, copper-based zeolite fiber layer and hydrophilic fiber layer has less cytotoxicity. In addition, if the surface of the copper-based zeolite in the copper-based zeolite fiber layer is modified with -NH₂ groups or -COO⁻ groups, it can be coordinated with copper ions with electron acceptors on the copper-based zeolite surface to lock Cu²⁺ , reduce the dissolution of Cu²⁺ and the possibility of interaction with cells; bacteria or viruses are adsorbed by the zeolite, and then the activity of viruses and bacteria is inhibited by the high concentration of copper ions on the surface of the copper zeolite. This technical feature not only further improves the cell survival rate, but also increases the efficiency of inactivating SARS-CoV-2.

### Example 5

The manufacturing method of the mask 5 of the present disclosure includes the following steps:
The mask includes a copper-based zeolite fiber layer, a hydrophobic fiber layer, a melt-blown non-woven fabric, a copper-based zeolite fiber layer, and a hydrophilic fiber layer. Among them, the manufacturing method of the copper-based zeolite fiber layer is as follows:
Preparing the zeolite precursor solution according to the following molar ratio of 8Na₂O:Al₂O₃:9SiO₂:180H₂O. 60 cotton fiber were twisted and spun into fiber threads. The zeolite precursor solution and the fiber threads were mixed uniformly and heat treated at 110°C for 36 h to obtain Y-type zeolite fiber threads. After soaking in a 5 M copper sulfate solution for 3 times, each time for 3 h, rinsing with deionized water several times, fiber threads containing copper-based zeolite are obtained.

The copper-based zeolite-containing fiber threads are used as warp and weft to knit into a copper-based zeolite fiber layer.

The Si/Al ratio of the copper-based zeolite is 2; Cu²⁺ accounts for 20% of the charge-balanced metal ion content outside the framework of the copper-based zeolite; and the mass fraction of copper in the fiber layer of the copper-based zeolite is 2 wt%.

The copper-based zeolite fiber layer, hydrophobic fiber layer, melt-blown non-woven fabric, copper-based zeolite fiber layer, and hydrophilic fiber layer are sequentially heat-sealed to prepare the cover of the mask 5 of the present disclosure. In the cytotoxicity test, the dissolution of copper ions from the cover of mask 5 was 0.98 ppm, and the cell survival rate was 83%; in the anti-SARS-CoV-2 test, the virus inactivation rate was 99.92%.

### Examples 6-11

Masks 6-11 include a copper-based zeolite fiber layer, a hydrophobic fiber layer, a copper-based zeolite fiber layer, and a hydrophilic fiber layer. The described manufacturing steps are the same as in Example 1, except that the composition of the copper-based zeolite fiber layer is changed to prepare the masks of Examples 6-11 (Tables 4 and 5). The details are as follows.

**Table 4 Composition of copper-based zeolite fiber layers of mask materials**

| Number | Example | Copper-based zeolite particle size D90/µm | Quality ratio of copper in copper-based zeolite fibers/wt% | The ratio of Copper accounts for a metal ion of the outer balanced charge of copper-based zeolite /% | Si/Al ratio of copper-based zeolite |
|---|---|---|---|---|---|
| 1 | Example 6 | 0.5 | 0.02 | 5 | 1.5 |
| 2 | Example 7 | 1 | 0.5 | 10 | 1.8 |
| 3 | Example 8 | 5 | 1 | 15 | 2 |
| 4 | Example 9 | 10 | 1.5 | 20 | 2.5 |
| 5 | Example 10 | 15 | 2 | 30 | 3 |
| 6 | Example 11 | 20 | 4 | 40 | 5 |

**Table 5 cytotoxicity and antiviral, antibacterial performance of mask materials**

| | Cell survival rate/% | Antibacterial rate (*E. coli*)/% | Antibacterial rate (*Pneumoniae*)/% | Antibacterial rate (*Staphylococcus aureus*)/% | Antiviral rate (SARS-CoV-2)/% |
|---|---|---|---|---|---|
| Example6 | 97 | 95 | 96 | 95 | 99.95 |
| Example 7 | 93 | 98 | 98 | 97 | 99.89 |
| Example 8 | 90 | >99.99 | >99.99 | >99.99 | 99.98 |
| Example 9 | 92 | >99.99 | >99.99 | >99.99 | 99.99 |
| Example 10 | 80 | >99.99 | >99.99 | >99.99 | 99.99 |
| Example 11 | 70 | >99.99 | >99.99 | >99.99 | 99.99 |

The above embodiments are only used to illustrate the present disclosure and not to limit the scope of the present disclosure. Although Examples 1-11 use masks as an example to illustrate the manufacturing method, structural characteristics, biological safety (such as cytotoxicity), and antibacterial and antiviral performance of the medical materials of the present disclosure, the technical solutions of the present disclosure can also be applied to various protective equipment. The protective equipment refers to wearable product medical staff occupational safety, and the wearables are selected from the group consisting of protective clothing, face screens, gloves, shoes, boots, caps, earmuffs. In addition, it should be understood that after reading the teachings of the present disclosure, those skilled in the art can make various changes or modifications to the present disclosure, which also fall within the scope defined by the appended claims of the present application.

## Claims

1. A multilayer copper-based zeolite fiber medical material, wherein the medical material comprises a four-layer structure, including
an outermost layer,
a first intermediate layer,
a second intermediate layer, and
an innermost layer in sequence;
wherein the outermost layer and the second intermediate layer are copper-based zeolite fiber layers;
the first intermediate layer is a hydrophobic fiber layer, and the innermost layer is a hydrophilic fiber layer; the hydrophilic fiber layer is composed of hydrophilic fibers; the hydrophilic fiber means that the macromolecular chain of the fiber has polar groups; the polar groups are selected from the group consisting of -OH, -NH2, C=O; the hydrophobic fiber is selected from the group consisting of polyethylene, polypropylene, polyester, modified polyethylene, modified polypropylene, and modified polyester;
a pore size of the copper-based zeolite fiber layer of the outermost layer is greater than that of the hydrophobic fiber layer of the first intermediate layer;
the copper-based zeolite fiber layer contains copper-based zeolite and fiber threads; the fiber threads are formed by twisting fibers;
the copper-based zeolite is independently dispersed on the fiber surface, and a mass of the copper-based zeolite decreases in a gradient from outside to inside along a radial orientation of the fiber thread; wherein the independent dispersion means that the minimum distance between the copper-based zeolite microscopic particles and the closest copper-based zeolite microscopic particles is greater than or equal to one-half of the sum of the particle diameters of the two copper-based zeolite microscopic particles, that is: d≥r₁+r₂; where r₁, r₂ respectively represent one-half of the particle size of two adjacent copper-based zeolite microscopic particles; d represents the minimum distance between two adjacent copper-based zeolite microscopic particles;
preferably, the copper-based zeolite contains copper element with electron acceptor; more preferably, the electron acceptor refers to the copper element of the copper-based zeolite is capable of accepting electrons from the surface groups of the copper-based zeolite;
preferably, the surface or the inside/outside of the zeolite framework of the copper-based zeolite contains copper ions; more preferably, the copper ions are selected from the group consisting of Cu¹⁺ and Cu²⁺;
preferably, the copper-based zeolite includes a group containing a lone electron pair and/or a group containing a conjugation effect;
preferably, the copper-based zeolite contains a -COOH group and/or a -COO- group;
preferably, the copper-based zeolite includes N group containing a lone pair of electrons; more preferably, the N group containing a lone pair of electrons comprises -NH₂ group;
preferably, the particle size D90 of the copper-based zeolite is 0.5 to 20µm; more preferably, the particle size D90 of the copper-based zeolite is 1 to 15µm; more preferably, the particle size D90 of the copper-based zeolite is 5 to 10µm;
preferably, the mass fraction of copper element in the copper -based zeolite fiber layer is 0.02 to 4wt%; more preferably, the mass fraction of copper element in the copper-based zeolite fiber layer is 0.5 to 2wt%; more preferably, the mass fraction of copper element in the copper-based zeolite fiber layer is 1 to 1.5wt%.

2. The multilayer copper-based zeolite fiber medical material of claim 1, wherein a melt-blown non-woven fabric layer is added between the first intermediate layer and the second intermediate layer; preferably, the melt brown non-woven fabric layer comprises any one or more of polyethylene, polypropylene, polyester, modified polyethylene, modified polypropylene, and modified polyester.

3. The multilayer copper-based zeolite fiber medical material of any one of claims 1-2, wherein the pore size of the copper-based zeolite fiber layer of the second intermediate layer is greater than that of the hydrophilic fiber layer of the innermost layer.

4. The multilayer copper-based zeolite fiber medical material of any one of claims 1-3, wherein the Cu²⁺ accounts for 5% to 40% of the charge-balanced metal ion content outside the framework of the copper-based zeolite; preferably, Cu²⁺ accounts for 10% to 30% of the charge-balanced metal ion content outside the framework of the copper-based zeolite; preferably, the Cu²⁺ accounts for 15% to 20% of the charge-balanced metal ion content outside the framework of the copper-based zeolite.

5. The multilayer copper-based zeolite fiber medical material of any one of claims 1-4, wherein the Si/ Al ratio of the copper-based zeolite is 1.5 to 5; preferably, the copper-based zeolite The Si/Al ratio of the zeolite is 1.8 to 3; preferably, the Si/Al ratio of the copper-based zeolite is 2 to 2.5.

6. The multilayer copper-based zeolite fiber medical material of any one of claims 1-5, wherein the copper-based zeolite independently dispersed on the fiber surface, and each copper-based zeolite microscopic particle has its own independent boundary.

7. The multilayer copper-based zeolite fiber medical material of any one of claims 1-6, wherein the copper-based zeolite is composed of zeolite nanoparticles.

8. The multilayer copper-based zeolite fiber medical material of any one of claims 1-7, wherein the copper-based zeolite is selected from the group consisting of X-type zeolite, Y-type zeolite, A-type zeolite, chabazite, L-type zeolite and P-type zeolite.

9. The multilayer copper-based zeolite fiber medical material of any one of claims 1-8, wherein the fibers of the copper-based zeolite fiber layer are selected from the group consisting of rayon fibers, acetate fibers, carboxymethyl cellulose, bamboo fibers, cotton fibers, wood fibers, polypropylene fibers, and polyethylene fibers.

10. A manufacturing method of a multilayer copper-based zeolite fiber medical material, wherein the following steps comprises:
1) preparing a hydrophobic fiber layer and a hydrophilic fiber layer;
2) preparing a copper-based zeolite fiber layer, wherein the manufacturing method of the copper-based zeolite fiber layer comprises:
(a) twisting the fibers to form a fiber thread, and synthesizing a fiber thread containing copper-based zeolite by using the fiber thread as a scaffold for zeolite nucleation and growth; wherein the content of copper-based zeolite decreases along the radial orientation of the fiber thread from the outside to the inside in a gradient;
(b) forming a copper-based zeolite fiber layer by weaving the copper-based zeolite-containing fiber threads as warp and weft;
3) preparing a four-layer antibacterial and antiviral medical material in the order of a copper-based zeolite fiber layer, a hydrophobic fiber layer, a copper-based zeolite fiber layer, and a hydrophilic fiber layer; wherein a pore size of the outermost copper-based zeolite fiber layer is greater than that of the hydrophobic fiber layer; the hydrophilic fiber layer is composed of hydrophilic fibers; the hydrophilic fiber means that the macromolecular chain of the fiber has polar groups; the polar group is selected from the group consisting of -OH, -NH2, C=O; the hydrophobic fibers are selected from the group consisting of polyethylene, polypropylene, polyester, modified polyethylene, modified polypropylene, and modified polyester;preferably, a melt-blown non-woven fabric layer is added between the hydrophobic fiber layer and the hydrophilic fiber layer; more preferably, the melt-blown non-woven fabric layer comprises any one or more of polypropylene, polyester, modified polyethylene, modified polypropylene, and modified polyester.

11. A protective equipment, wherein the protective equipment comprises the multilayer copper-based zeolite fiber medical material according to any one of claims 1-9 or the multilayer copper-based zeolite fiber medical material obtained by the manufacturing method according to claim 10.

12. The protective equipment of claim 11, wherein the protective equipment refers to wearables used for safety protection from virus or bacteria, and the wearables are selected from the group consisting of masks, protective masks, protective clothing, face screens, gloves, shoes, boots, hats, and earmuffs.

## Patentansprüche

1. Mehrschichtiges medizinisches Zeolithfasermaterial auf Kupferbasis, wobei das medizinische Material eine vierschichtige Struktur aufweist, in Folge umfassend:
eine äußerste Schicht,
eine erste Zwischenschicht,
eine zweite Zwischenschicht, und
eine innerste Schicht;
wobei die äußerste Schicht und die zweite Zwischenschicht Zeolithfaserschichten auf Kupferbasis sind;
wobei die erste Zwischenschicht eine hydrophobe Faserschicht ist und die innerste Schicht eine hydrophile Faserschicht ist; wobei die hydrophile Faserschicht aus hydrophilen Fasern besteht; wobei die hydrophile Faser bedeutet, dass die makromolekulare Kette der Faser polare Gruppen aufweist; wobei die polaren Gruppenaus der Gruppe bestehend aus -OH, -NH₂, C=O ausgewählt sind; wobei die hydrophobe Faser aus der Gruppe bestehend aus Polyethylen, Polypropylen, Polyester, modifiziertem Polyethylen, modifiziertem Polypropylen und modifiziertem Polyester ausgewählt ist;
wobei eine Porengröße der Zeolithfaserschicht auf Kupferbasis der äußersten Schicht größer als die der hydrophoben Faserschicht der ersten Zwischenschicht ist;
wobei die Zeolithfaserschicht auf Kupferbasis Zeolith auf Kupferbasis und Faserfäden enthält; wobei die Faserfäden durch Verdrillen von Fasern gebildet werden;
wobei der Zeolith auf Kupferbasis unabhängig auf der Faseroberfläche verteilt ist und eine Masse des Zeoliths auf Kupferbasis in einem Gradienten von außen nach innen entlang einer radialen Ausrichtung des Faserfadens abnimmt; wobei die unabhängige Verteilung bedeutet, dass der Mindestabstand zwischen der Mikropartikel des Zeoliths auf Kupferbasis und den nächstgelegenen Mikropartikel des Zeoliths auf Kupferbasis größer als oder gleich der Hälfte der Summe der Partikeldurchmesser der beiden Mikropartikel des Zeoliths auf Kupferbasis ist, d.h.: d≥ r1+r2; wobei r1, r2 jeweils die Hälfte der Partikelgröße von zwei benachbarten Mikropartikel des Zeoliths auf Kupferbasis darstellen; wobei d den Mindestabstand zwischen zwei benachbarten Mikropartikel des Zeoliths auf Kupferbasis bezeichnet;
wobei vorzugsweise der Zeolith auf Kupferbasis ein Kupferelement mit einem Elektronenakzeptor enthält; wobei sich der Elektronenakzeptor noch bevorzugter auf das Kupferelement des Zeoliths auf Kupferbasis bezieht, das in der Lage ist, Elektronen von den Oberflächengruppen des Zeoliths auf Kupferbasis aufzunehmen;
wobei vorzugsweise die Oberfläche oder die Innen-/Außenseite des Zeolithrahmens des Zeoliths auf Kupferbasis Kupferionen enthält; wobei die Kupferionen noch bevorzugter aus der Gruppe bestehend aus Cu¹⁺ und Cu²⁺ ausgewählt sind;
wobei der Zeolith auf Kupferbasis vorzugsweise eine Gruppe, die ein einsames Elektronenpaar enthält, und/oder eine Gruppe, die einen Konjugationseffekt aufweist, umfasst;
wobei der Zeolith auf Kupferbasis vorzugsweise eine -COOH-Gruppe und/oder eine -COO-Gruppe enthält;
wobei der Zeolith auf Kupferbasis vorzugsweise eine N-Gruppe umfasst, die ein einsames Elektronenpaar enthält; wobei noch bevorzugter die N-Gruppe, die ein einsames Elektronenpaar enthält, eine -NH₂-Gruppe umfasst;
wobei vorzugsweise die Partikelgröße D90 des Zeoliths auf Kupferbasis 0,5 bis 20 µm beträgt; wobei noch bevorzugter die Partikelgröße D90 des Zeoliths auf Kupferbasis 1 bis 15 µm beträgt; wobei noch bevorzugter die Partikelgröße D90 des Zeoliths auf Kupferbasis 5 bis 10 µm beträgt;
wobei der Massenanteil des Kupferelements in der Zeolithfaserschicht auf Kupferbasis vorzugsweise 0,02 bis 4 Gew.-% beträgt; wobei der Massenanteil des Kupferelements in der Zeolithfaserschicht auf Kupferbasis noch bevorzugter 0,5 bis 2 Gew.-% beträgt; und wobei der Massenanteil des Kupferelements in der Zeolithfaserschicht auf Kupferbasis noch bevorzugter 1 bis 1,5 Gew.-% beträgt.

2. Mehrschichtiges medizinisches Zeolithfasermaterial auf Kupferbasis nach Anspruch 1, wobei zwischen der ersten Zwischenschicht und der zweiten Zwischenschicht eine schmelzgeblasene Vliesstoffschicht hinzugefügt wird; wobei vorzugsweise die schmelzgeblasene Vliesstoffschicht eines oder mehrere der folgenden Materialien umfasst; Polyethylen, Polypropylen, Polyester, modifiziertes Polyethylen, modifiziertes Polypropylen und modifizierter Polyester.

3. Mehrschichtiges medizinisches Zeolithfasermaterial auf Kupferbasis nach einem der Ansprüche 1 bis 2, wobei die Porengröße der Zeolithfaserschicht auf Kupferbasis der zweiten Zwischenschicht größer ist als die der hydrophilen Faserschicht der innersten Schicht.

4. Mehrschichtiges medizinisches Zeolithfasermaterial auf Kupferbasis nach einem der Ansprüche 1 bis 3, wobei das Cu²⁺ 5 % bis 40 % des ladungsausgeglichenen Metallionengehalts außerhalb des Zeolithrahmens des Zeoliths auf Kupferbasis ausmacht; wobei vorzugsweise das Cu²⁺ 10 % bis 30 % des ladungsausgeglichenen Metallionengehalts außerhalb des Zeolithrahmens des Zeoliths auf Kupferbasis ausmacht; wobei vorzugsweise das Cu²⁺ 15 % bis 20 % des ladungsausgeglichenen Metallionengehalts außerhalb des Zeolithrahmens des Zeoliths auf Kupferbasis ausmacht.

5. Mehrschichtiges medizinisches Zeolithfasermaterial auf Kupferbasis nach einem der Ansprüche 1 bis 4, wobei das Si/Al-Verhältnis des Zeoliths auf Kupferbasis 1,5 bis 5 beträgt; wobei das Si/Al-Verhältnis des Zeoliths auf Kupferbasis vorzugsweise 1,8 bis 3 beträgt; wobei das Si/Al-Verhältnis des Zeoliths auf Kupferbasis vorzugsweise 2 bis 2,5 beträgt.

6. Mehrschichtiges medizinisches Zeolithfasermaterial auf Kupferbasis nach einem der Ansprüche 1 bis 5, wobei der Zeolith auf Kupferbasis unabhängig voneinander auf der Faseroberfläche verteilt ist und jede Mikropartikel des Zeoliths auf Kupferbasis seine eigene unabhängige Grenze hat.

7. Mehrschichtiges medizinisches Zeolithfasermaterial auf Kupferbasis nach einem der Ansprüche 1 bis 6, wobei das Zeolith auf Kupferbasis aus Zeolithnanopartikeln besteht.

8. Mehrschichtiges medizinisches Zeolithfasermaterial auf Kupferbasis nach einem der Ansprüche 1-7, wobei der Zeolith auf Kupferbasis aus der Gruppe bestehend aus X-Typ-Zeolith, Y-Typ-Zeolith, A-Typ-Zeolith, Chabazit, L-Typ-Zeolith und P-Typ-Zeolith ausgewählt ist.

9. Mehrschichtiges medizinisches Zeolithfasermaterial auf Kupferbasis nach einem der Ansprüche 1-8, wobei die Fasern der Zeolithfaserschicht auf Kupferbasis aus der Gruppe bestehend aus Rayonfasern, Acetatfasern, Carboxymethylcellulose, Bambusfasern, Baumwollfasern, Holzfasern, Polypropylenfasern und Polyethylenfasern ausgewählt sind.

10. Verfahren zur Herstellung eines mehrschichtigen medizinischen Zeolithfasermaterials auf Kupferbasis, wobei es die folgenden Schritte umfassen:
1) Vorbereiten einer hydrophoben Faserschicht und einer hydrophilen Faserschicht;
2) Vorbereiten einer Zeolithfaserschicht auf Kupferbasis, wobei das Vorbereitenverfahren der Zeolithfaserschicht auf Kupferbasis Folgendes umfasst:
(a) Verdrehen der Fasern, um einen Faserfaden zu bilden, und Synthetisieren eines Faserfadens, der Zeolith auf Kupferbasis enthält, indem der Faserfaden als Gerüst für die Keimbildung und das Wachstum des Zeoliths verwendet wird; wobei der Gehalt an Zeolith auf Kupferbasis entlang der radialen Ausrichtung des Faserfadens von außen nach innen in einem Gradienten abnimmt;
(b) Bilden einer Zeolith-Faserschicht auf Kupferbasis durch Verweben der kupferbasierten zeolithhaltigen Faserfäden als Kett- und Schussfaden;
3) Vorbereiten eines vierschichtigen antibakteriellen und antiviralen medizinischen Materials in der Reihenfolge einer Zeolithfaserschicht auf Kupferbasis, einer hydrophoben Faserschicht, einer Zeolithfaserschicht auf Kupferbasis und einer hydrophilen Faserschicht; wobei die Porengröße der äußersten Zeolithfaserschicht auf Kupferbasis größer als die der hydrophoben Faserschicht ist; wobei die hydrophile Faserschicht aus hydrophilen Fasern besteht; wobei die hydrophile Faser bedeutet, dass die makromolekulare Kette der Faser polare Gruppen aufweist; wobei die polaren Gruppen aus der Gruppe bestehend aus -OH, -NH₂, C=O ausgewählt sind; wobei die hydrophobe Fasern aus der Gruppe bestehend aus Polyethylen, Polypropylen, Polyester, modifiziertem Polyethylen, modifiziertem Polypropylen und modifiziertem Polyester ausgewählt sind; wobei vorzugsweise zwischen der ersten Zwischenschicht und der zweiten Zwischenschicht eine schmelzgeblasene Vliesstoffschicht hinzugefügt wird; wobei noch vorzugsweise die schmelzgeblasene Vliesstoffschicht eines oder mehrere der folgenden Materialien umfasst; Polyethylen, Polypropylen, Polyester, modifiziertes Polyethylen, modifiziertes Polypropylen und modifizierter Polyester.

11. Schutzausrüstung, wobei die Schutzausrüstung das mehrschichtige medizinische Zeolithfasermaterial auf Kupferbasis nach einem der Ansprüche 1 bis 9 oder das mehrschichtige medizinische Zeolithfasermaterial auf Kupferbasis umfasst, das durch das Verfahren nach Anspruch 10 erhalten wurde.

12. Schutzausrüstung nach Anspruch 11, wobei sich die Schutzausrüstung auf Verschleißteile bezieht, die zum Schutz vor Viren oder Bakterien verwendet werden, und wobei die Verschleißteile aus der Gruppe ausgewählt sind, die aus Masken, Schutzmasken, Schutzkleidung, Gesichtsschutz, Handschuhen, Schuhen, Stiefeln, Mützen und Ohrenschützern besteht.

## Revendications

1. Matériau médical multicouche de fibre zéolitique à base de cuivre, dans lequel le Matériau médical comprend une structure à quatre couches, comprenant dans l'ordre
une couche la plus extérieure,
une première couche intermédiaire,
une deuxième couche intermédiaire, et
une couche la plus intérieure ;
dans lequel où la couche la plus extérieure et la deuxième couche intermédiaire sont des couches de fibre zéolitique à base de cuivre ;
la première couche intermédiaire est une couche de fibre hydrophobe, et la couche la plus intérieure est une couche de fibre hydrophile ; la couche de fibre hydrophile est composée de fibres hydrophiles ; la fibre hydrophile signifie que la chaîne macromoléculaire de la fibre contient des groupes polaires ; les groupes polaires sont choisis dans le groupe constitué de -OH, -NH2 et C=O ; la fibre hydrophobe est choisie dans le groupe constitué du polyéthylène, du polypropylène, du polyester, du polyéthylène modifié, du polypropylène modifié, et du polyester modifié ;
la taille de pores de la couche de fibre zéolitique à base de cuivre de la couche la plus extérieure est supérieure à celle de la couche de fibre hydrophobe de la première couche intermédiaire ;
la couche de fibre zéolitique à base de cuivre contient de la zéolite à base de cuivre et des fils fibreux ; les fils fibreux sont formés en tordant des fibres ;
la zéolite à base de cuivre est dispersée indépendamment sur la surface de la fibre, et une masse de la zéolite à base de cuivre diminue progressivement de l'extérieur vers l'intérieur au long d'une orientation radiale du fil fibreux ; dans lequel la dispersion indépendante signifie que la distance minimale entre les particules microscopiques de la zéolite à base de cuivre et les particules microscopiques de la zéolite à base de cuivre les plus proches est supérieure ou égale à la moitié de la somme des diamètres de particule des deux particules microscopiques de la zéolite à base de cuivre, c'est-à-dire : d ≥ r₁+ r₂ ; où r₁ et r₂ représentent respectivement la moitié de la taille de particules des deux particules microscopiques de la zéolite à base de cuivre adjacentes ; d représente la distance minimale entre deux particules microscopiques de la zéolite à base de cuivre adjacentes ;
de préférence, la zéolite à base de cuivre contient des éléments de cuivre avec un accepteur d'électrons ; de plus préférence, l'accepteur d'électrons réfère à l'élément de cuivre capable d'accepter des électrons des groupes de surface de la zéolite à base de cuivre ;
de préférence, la surface ou l'intérieur/l'extérieur de la structure zéolitique de la zéolite à base de cuivre contient des ions de cuivre ; de plus préférence, les ions de cuivre sont choisis dans le groupe constitué de Cu¹⁺ et Cu²⁺ ;
de préférence, la zéolite à base de cuivre comprend un groupe contenant un doublet non liant et/ou un groupe à un effet de conjugaison ;
de préférence, la zéolite à base de cuivre contient un groupe -COOH et/ou un groupe -COO- ;
de préférence, la zéolite à base de cuivre comprend un groupe nitrile contenant un doublet non liant ; de plus préférence, le groupe nitrile contenant un doublet non liant comprend un groupe -NH₂ ;
de préférence, la taille des particules D90 de la zéolite à base de cuivre est comprise entre 0,5 et 20 µm ; de plus préférence, la taille des particules D90 de la zéolite à base de cuivre est comprise entre 1 et 15 µm ; de plus préférence, la taille des particules D90 de la zéolite à base de cuivre est comprise entre 5 et 10 µm ;
de préférence, la fraction massique de l'élément de cuivre dans la couche de fibre zéolitique à base de cuivre est comprise entre 0,02% et 4% ; de plus préférence, la fraction massique de l'élément de cuivre dans la couche de fibre zéolitique à base de cuivre est comprise entre 0,5% et 2% ; de plus préférence, la fraction massique de l'élément de cuivre dans la couche de fibre zéolitique à base de cuivre est comprise entre 1% et 1,5%.

2. Matériau médical multicouche de fibre zéolitique à base de cuivre selon la revendication 1, dans lequel une couche de tissu non-tissé soufflée est ajoutée entre la première couche intermédiaire et la deuxième couche intermédiaire ; de préférence, la couche de tissu non-tissé soufflée comprend un ou plusieurs des composés suivants : le polyéthylène, le polypropylène, le polyester, le polyéthylène modifié, le polypropylène modifié et le polyester modifié.

3. Matériau médical multicouche de fibre zéolitique à base de cuivre selon l'une des revendications 1 à 2, dans lequel la taille des pores de la couche de fibre zéolitique à base de cuivre de la deuxième couche intermédiaire est supérieure à celle de la couche de fibre hydrophile de la couche la plus intérieure.

4. Matériau médical multicouche de fibre zéolitique à base de cuivre selon l'une des revendications 1 à 3, dans lequel le Cu²⁺ représente 5% à 40% de la teneur en ions métaux à charge équilibrée outre la structure de la zéolite à base de cuivre ; de préférence, le Cu²⁺ représente 15% à 20% de la teneur en ions outre la structure de la zéolite à base de cuivre.

5. Matériau médical multicouche de fibre zéolitique à base de cuivre selon l'une des revendications 1 à 4, dans lequel le rapport Si/Al de la zéolite à base de cuivre est compris entre 1,5 et 5 ; de préférence, le rapport Si/Al de la zéolite à base de cuivre est compris entre 1,8 et 3 ; de préférence, le rapport Si/Al de la zéolite à base de cuivre est compris entre 2 et 2,5.

6. Matériau médical multicouche de fibre zéolitique à base de cuivre selon l'une des revendications 1 à 5, dans lequel la zéolite à base de cuivre est dispersée indépendamment sur la surface de la fibre, et chaque particule microscopique de zéolite à base de cuivre a sa propre bordure indépendante.

7. Matériau médical multicouche de fibre zéolitique à base de cuivre selon l'une des revendications 1 à 6, dans lequel la zéolite à base de cuivre est composé de nanoparticules zéolitiques.

8. Matériau médical multicouche de fibre zéolitique à base de cuivre selon l'une des revendications 1 à 7, dans lequel la zéolite à base de cuivre est choisie dans le groupe constitué de la zéolite de type X, de la zéolite de type Y, de la zéolite de type A, de la chabazite, de la zéolite de type L et de la zéolite de type P.

9. Matériau médical multicouche de fibre zéolitique à base de cuivre selon l'une des revendications 1 à 8, dans lequel les fibres de zéolite à base de cuivre sont choisies dans le groupe constitué des fibres de rayonne, des fibres d'acétate, de la carboxyméthylcellulose, des fibres de bambou, des fibres de coton, des fibres de bois, des fibres de polypropylène et des fibres de polyéthylène.

10. Procédé de fabrication d'un matériau médical multicouche de fibre zéolitique à base de cuivre, dans lequel le procédé comprend les étapes suivantes :
1) préparer une couche de fibre hydrophobe et une couche de fibre hydrophile ;
2) préparer une couche de fibre à base de cuivre, dans lequel le procédé de fabrication de la couche de zéolite à base de cuivre comprend :
(a) tordre les fibres pour former un fil fibreux, et synthétiser un fil fibreux contenant de la zéolite à base de cuivre en utilisant le fil fibreux comme un échafaudage pour la nucléation et la croissance de zéolite ; dans lequel le teneur de zéolite à base de cuivre diminue progressivement le long de l'orientation radiale du fil fibreux de l'extérieur vers l'intérieur ;
(b) former une couche de fibre zéolitique à base de cuivre en tissant la zéolite à base de cuivre contenant des fils fibreux en tant que chaîne et trame ;
3) préparer un matériau médical antibactérien et antiviral à quatre couches dans l'ordre suivant : une couche de fibre zéolitique à base de cuivre, une couche de fibre hydrophobe, une couche de fibre zéolitique à base de cuivre et une couche de fibre hydrophile ; dans lequel la taille des pores de la couche de fibre zéolitique à base de cuivre la plus extérieure est supérieure à celle de la couche de fibre hydrophobe ; la couche de fibre hydrophile est composé de fibres hydrophiles ; la fibre hydrophile signifie que la chaîne macromoléculaire de la fibre contient des groupes polaires ; le groupe polaire est choisi dans le groupe constitué de -OH, -NH₂, C=0 ; les fibres hydrophobes sont choisies dans le groupe constitué de polyéthylène, polypropylène, polyester, polyéthylène modifié, polypropylène modifié et polyester modifié ; de préférence, une couche de tissu non-tissé soufflée est ajoutée entre la couche de fibre hydrophobe et la couche de fibre hydrophile ; de plus préférence, la couche de tissu non-tissé soufflée comprend un ou plusieurs des composés suivants : le polypropylène, le polyester, le polyéthylène modifié, le polypropylène modifié et le polyester modifié.

11. Équipement de protection, dans lequel l'équipement de protection comprend le Matériau médical multicouche de fibre zéolitique à base de cuivre selon l'une des revendications 1 à 9 ou le Matériau médical multicouche de fibre zéolitique à base de cuivre obtenu par le procédé de fabrication selon la revendication 10.

12. Équipement de protection selon la revendication 11, dans lequel l'équipement de protection réfère aux articles portables utilisés pour la protection de sécurité contre les virus ou les bactéries, les articles portables sont choisis dans le groupe constitué des masques, des masques de protection, des vêtements de protection, des écrans faciaux, des gants, des chaussures, des bottes, des chapeaux et des cache-oreilles.
